(19) Europäisches Patentamt
European Patent Office
Office européen des brevets

(11) Veröffentlichungsnummer: **0 011 874**
**B1**

(12) **EUROPÄISCHE PATENTSCHRIFT**

(45) Veröffentlichungstag der Patentschrift: **12.05.82**

(51) Int. Cl.³: **C 07 C 11/04**, C 07 C 4/04 // C23C1/08, C23C1/10, C23C11/02

(21) Anmeldenummer: **79104817.6**

(22) Anmeldetag: **01.12.79**

# (54) Verfahren zur Herstellung von Ethylen durch Hydropyrolyse.

(30) Priorität: **04.12.78 DE 2852314**

(43) Veröffentlichungstag der Anmeldung: **11.06.80 Patentblatt 80/12**

(45) Bekanntmachung des Hinweises auf die Patenterteilung: **12.05.82 Patentblatt 82/19**

(84) Benannte Vertragsstaaten: **BE DE FR GB NL**

(56) Entgegenhaltungen:
**AT-B-153 171**
**AT-B-189 008**
**DE-A1-2 527 316**
**DE-A1-2 528 554**
**DE-A1-2 553 413**
**DE-C-630 872**
**US-A-2 343 866**
**US-A-3 778 488**
**US-A-3 798 283**

(73) Patentinhaber: **HOECHST AKTIENGESELLSCHAFT, Postfach 80 03 20, D-6230 Frankfurt/Main 80 (DE)**

(72) Erfinder: **Vogt, Wilhelm, Dr., Bellerstrasse 74, D-5030 Hürth (DE)**
Erfinder: **Glaser, Hermann, Magdalenenweg 16, D-5042 Erftstadt (DE)**

**EP 0 011 874 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Ethylen durch Hydropyrolyse

Die vorliegende Erfindung betrifft ein Verfahren zur Herstellung von Ethylen durch Hydropyrolyse eines Kohlenwasserstoffe, Wasserstoff, Kohlenmonoxid, Kohlendioxid und Wasserdampf enthaltenden Gasgemisches in einer Reaktionszone bei Temperaturen von über 800°C bis zu 1000°C.

Es ist ein Verfahren zur Herstellung von Ethan und/oder Ethylen bekannt, nach welchem ein aus Kohlenwasserstoffen, Kohlenmonoxid, Kohlendioxid, Wasser und Wasserstoff bestehendes Reaktionsgemisch, welches durch Umsetzung von Wasserstoff und Kohlenmonoxid im Molverhältnis von 1 : 1 bis 5 : 1 an einem eisen-, kobalt-, nickel- oder rutheniumhaltigen Fischer-Tropsch-Katalysator erhalten wurde, in einer Hydropyrolysezone bei Temperaturen von 600 bis 900°C und Drucken von mindestens 5 bar sowie mit Verweilzeiten von 0,1 bis 60 Sekunden pyrolisiert wird und die $C_2$-Kohlenwasserstoffe aus dem aus der Hydropyrolysezone abströmenden Gasgemisch abgetrennt werden. Dabei kann die Hydropyrolyse in einem Reaktor durchgeführt werden, welcher im Inneren einen hitzebeständigen Stahlzylinder aufweist, mit welchem das Reaktionsgemisch in Berührung kommt.

Beim Verfahren zur Herstellung von Acetylen durch Pyrolyse von Kohlenwasserstoffen nach der US-A-2 343 866 werden zunächst die Kohlenwasserstoffe durch eine aus einer Eisenlegierung bestehende und von außen beheizte Reaktionskammer geleitet und dabei in kurzer Zeit auf 800 bis 1000°C erhitzt. Dabei ist es möglich, eine Eisenlegierung zu benutzen, welche Chrom oder Silicium oder Aluminium oder Wolfram oder Molybdän oder Mangan enthält. Die erhitzten Kohlenwasserstoffe werden unmittelbar anschließend bei Temperaturen von 1000 bis 1600°C thermisch zersetzt, indem sie im Gegenstrom mit heißen, durch Verbrennen von Kohlenwasserstoffen erhaltenen Verbrennungsprodukten in Berührung gebracht werden, wodurch die Kohlenwasserstoffe mindestens teilweise in Acetylen umgewandelt werden.

Aus der US-A-3 778 488 ist ein Verfahren zur oxidativen Dehydrogenation von organischen Verbindungen, insbesondere von Buten, bekannt, bei welchem die organische Verbindung in Gegenwart von Sauerstoff bei Temperaturen von 430 bis 650°C über einen Katalysator geleitet wird, welcher aus Eisen und mindestens einem weiteren Metall wie Kupfer oder Aluminium besteht, wobei die Oberfläche des Katalysators in die Oxide überführt ist.

Schließlich lassen sich nahtlose oder geschweißte Stahlrohre an ihrer Innenseite gemäß der AT-B-153 171 dadurch verkupfern, daß man in das Stahlrohr einen schraubenförmig gewickelten Kupferdraht einführt, wobei der Außendurchmesser seiner Windung gleich dem Innendurchmesser des Stahlrohres ist. Das so vorbereitete Rohr wird zweckmäßigerweise schräg gestellt durch einen Ofen geführt, welcher die Schmelztemperatur des Kupfers hat und unter reduzierender Atmosphäre steht.

Die Hydropyrolyse von Reaktionsgemischen, welche sich bei der katalytischen Reduktion von Kohlenmonoxid mit Wasserstoff, beispielsweise gemäß der DE-PS 2 546 587 und der DE-OS 2 704 575 oder der DE-OS 2 653 985 oder der DE-AS 2 653 986, bilden, ist nur möglich, wenn die Einstellung der Gleichgewichte der Reaktionen

$$H_2 + CO_2 \rightarrow H_2O + CO$$
$$-CH_2- + H_2O \rightarrow CO + 2\,H_2$$
$$-CH_2- + 2\,CO_2 \rightarrow 3\,CO + H_2O$$

bei der erforderlichen Temperatur von etwa 800 bis 950°C nicht erfolgt und wenn weder die Kohlenwasserstoffe noch das Kohlenmonoxid verfahrensbeeinträchtigende Mengen Ruß bilden. Die genannten Reaktionen laufen jedoch so langsam ab, daß sie sich bei der erforderlichen Verweilzeit von 0,02 bis 2 Sekunden auf die Ausbeute der bei der Hydropyrolyse gebildeten Olefine nicht merklich nachteilig auswirken, sofern diese Reaktionen nicht durch katalytische Einflüsse der Wandung des Pyrolysereaktors begünstigt wurden.

Weiterhin wurde gefunden, daß die Hydropyrolyse eines Kohlenwasserstoffe, Wasserstoff, Kohlenmonoxid, Kohlendioxid und Wasserdampf enthaltenden Gasgemisches 250 Stunden in Quarzrohren ohne Störung durch Rußabscheidung durchgeführt werden kann, während bei der Verwendung von Rohren, beispielsweise aus Eisen, temperaturbeständigen Stählen oder Nickel Rußbildung und/oder eine merkliche Zersetzung der Kohlenwasserstoffe erfolgt.

Es ist Aufgabe der vorliegenden Erfindung, ein großtechnisch durchführbares Pyrolyseverfahren zur Gewinnung von Ethylen aus einem Kohlenwasserstoffe, Wasserstoff, Kohlenmonoxid, Kohlendioxid und Wasser enthaltenden Gasgemisch anzugeben, bei welchem die Rußbildung, welche zur Verstopfung der Reaktionsrohre führt, unterbleibt und bei welchem darüber hinaus eine Zersetzung von Kohlenwasserstoffen weitgehend vermieden wird. Das wird erfindungsgemäß dadurch erreicht, daß das Gasgemisch in der Reaktionszone einen Druck von weniger als 5 bar aufweist und daß die vom Gasgemisch berührten metallischen Wände der Reaktionszone mindestens an ihrer Oberfläche Aluminium und/oder Kupfer enthalten.

Das Verfahren gemäß der Erfindung kann weiterhin wahlweise noch dadurch ausgestaltet sein, daß

a) das Gasgemisch einen Druck von 1,5 bis 4 bar aufweist,
b) die metallischen Wände der Reaktionszone aus einer aluminium- und/oder kupferhaltigen Stahlsorte bestehen,
c) auf die metallischen Wände der Reaktionszone Aluminium und/oder Kupfer aufgetragen sind,
d) das Auftragen von Aluminium und/oder Kupfer auf die metallischen Wände über die Gasphase mit Hilfe von Halogenverbindungen als Transportmittel bewirkt wird,
e) das Auftragen von Aluminium durch Inberührungbringen der metallischen Wände mit einer Schmelze aus Aluminium bewirkt wird,
f) dem Gasgemisch gasförmige Schwefelverbindungen zugesetzt sind.

Als Material für die Wände der Reaktionszone können Chrom- oder Chrom-Nickel-Stähle, welche zusätzlich Aluminium enthalten, verwendet werden, beispielsweise Aluchrom® (Werkstoffnummern 1.4765 und 1.4767 nach DIN 17 470, Juli 1963), Armco 18 SR® oder Kanthal®. Bei Verwendung von im wesentlichen aluminiumfreien Stählen ist es erforderlich, die dem Gasgemisch ausgesetzte Seite der Reaktionszone zu aluminieren. Ein zusätzliches Aluminieren ist auch bei aluminiumhaltigen Stählen von Vorteil, weil es die Wandkatalyse weiterhin abschwächt.

Zum Aluminieren der Innenwände der Reaktionszone kann man diese mit einer Mischung aus pulver- oder granalienförmigen Aluminium und einem porösen, mit Aluminium nicht reagierenden Trägermaterial, beispielsweise Aluminiumoxid, füllen und bei 700 bis 1000°C unter Spülen mit einer Mischung von Stickstoff und dampfförmigem Aluminiumchlorid tempern. Im Laufe von 2 bis 5 Stunden ist so viel Aluminium in die Wände diffundiert, daß die erforderliche katalytische Entaktivierung der metallischen Wände der Reaktionszone erreicht ist. Man kann zum Aluminieren den Reaktor auch mit einer Aluminiumschmelze von 700 bis 900°C füllen, welche man 2 bis 20 Stunden einwirken läßt. Eine besonders gute Herabsetzung der Wandkatalyse erreicht man, wenn man beim Aluminieren zusätzlich Kupfer — entweder in Pulver- oder Granalienform oder als Schmelze — hinzufügt.

Die katalytische Wirkung der Innenwände der Reaktionszone kann schließlich auch noch durch Zusatz schwefelhaltiger Verbindungen, beispielsweise $H_2S$, $CS_2$ und COS, zum Gasgemisch weiter herabgesetzt werden.

## Beispiele

Für alle Beispiele wurde ein Gasgemisch verwendet, welches durch Überleiten einer Mischung von Kohlenmonoxid und Wasserstoff im Volumen-Verhältnis 1 : 1 über einen Eisen, Kupfer und Kalium enthaltenden Trägerkatalysator (vergl. DE-AS 2 653 986) bei 290°C und 16 bar erhalten wurde. Eine typische Zusammensetzung dieses Gasgemisches ist in Tabelle 1, linke Spalte, angegeben.

Das Gasgemisch wurde jeweils ohne Abkühlung und nach Entspannung auf 1 bar in einen Hydropyrolyse-Reaktor eingefahren, dessen Rohre einen Innendurchmesser von 10 mm und eine beheizte Länge von 20 cm aufwiesen, wobei die verschiedenen Materialien für die Rohre in Tabelle 2 angegeben sind. Die Verweilzeit betrug, bezogen auf die Versuchsbedingungen (1 bar, 890°C), ca. 0,25 Sekunden. Die Volumenänderung variierte bei den in der Tabelle 2 angegebenen Beispielen 13 bis 22 um ca. −10%.

Wie aus der Tabelle 2 ersichtlich ist, sind die Rohrmaterialien, welche in den Vergleichsbeispielen 1 bis 12 verwendet wurden, ungeeignet, da sie die zur Verstopfung führende Rußbildung begünstigen und/oder eine Zunahme des CO-Gehaltes verursachen und/oder Reaktionsgase mit niedriger Ethylenkonzentration liefern.

Im Gegensatz dazu liefern die Rohrmaterialien, welche in den erfindungsgemäßen Beispielen 13 bis 22 verwendet wurden, Reaktionsgase mit hohen Ethylengehalten, ohne daß auch bei langen Laufzeiten eine Rußbildung erfolgt oder die CO-Konzentration, bezogen auf das eingefahrene Gasgemisch, spürbar zunimmt.

In Tabelle 1, rechts, ist eine typische Analyse eines Gases aufgeführt, welches gemäß dem erfindungsgemäßen Hydropyrolyse-Verfahren erhalten wurde.

In der Tabelle 2 bedeutet »a → b«, daß sich die Konzentration im Hydropyrolysegas während der Versuchsdauer von a auf b Vol.-% verändert hat.

Tabelle 1

| | Gasgemisch | | Hydropyrolysegas | | |
|---|---|---|---|---|---|
| | Zusammensetzung (Vol.-%) | Selektivität (%) | Zusammensetzung (Vol.-%) | Ausbeute (%; bezogen auf umgesetztes CO) | C-Prozente (bezogen auf Σ Kohlenwasserstoffe) |
| $H_2O$ | 2,85 | | 2,79 | | |
| $O_2$ | 0,59 | | 0,48 | | |
| $N_2$ | 0,24 | | 0,24 | | |
| $H_2$ | 26,42 | | 29,62 | | |
| CO | 14,33 | | 13,48 | | |
| $CO_2$ | 34,32 | 46,51 | 31,34 | 47,12 | – |
| $CH_4$ | 12,80 | 17,35 | 12,89 | 19,48 | 36,84 |
| $C_2H_2$ | <0,01 | | 0,37 | 1,12 | 2,11 |
| $C_2H_4$ | 0,59 | 9,27 | 6,84 | 20,67 | 39,10 |
| $C_2H_6$ | 2,32 | | 0,53 | 1,60 | 3,03 |
| $C_3$ | 2,41 | 9,80 | 0,59 | 2,67 | 5,06 |
| $C_4$ | 2,35 | 12,74 | 0,12 | 0,37 | 1,37 |
| $C_5$ | 0,54 | 3,66 | 0,26 | | |
| $C_6$ | 0,15 | 1,22 | 0,09 | | |
| $C_7$ | 0,04 | 0,38 | 0,25 | 6,76 | 12,78 |
| $C_8$ | <0,02 | <0,22 | 0,07 | | |
| $C_{8+}$ | <0,02 | ~0,22 | ~0,04 | | |

Tabelle 2

Vergleichsbeispiele

| Rohrmaterial | Laufzeit (h) | CO-Zunahme (Vol.-%) | Gehalt in Vol.-% an $CH_4$ | $C_2H_4$ | Bemerkungen |
|---|---|---|---|---|---|
| 1. Eisen | 14 | 18 → 0 | 17 | 4,2 | verstopft durch Ruß |
| 2. Eisen mit S behandelt | 24 | 4 → 0 | 16 → 9 | 5,9 → 0,4 | |
| 3. Eisen mit P behandelt | 4 | 5 → 20 | 13 | 6 → 0,7 | |
| 4. Eisen mit Kieselsäureester behandelt | 6 | 10 | 15 | 6 → 3,5 | |
| 5. dito 4; 4 h geglüht | 18 | 16 → 2 | 11 → 8 | 2,8 → 0,4 | |
| 6. dito 5; sulfidiertes Gasgemisch | 3 | 5 → 15 | 12 → 17 | 5,5 → 1,8 | |
| 7. Eisen mit $H(AuCl_4)$ vergoldet | 22 | 30 | 9 | 1,5 | |
| 8. Stahl, Werkstoff Nr. 1.4016 (Cekas®) | 19 | 3 | 12,8 | 5,9 | Rußbildung |
| 9. dito 8; sulfidiertes Gasgemisch | 21 | 1 | 15,2 | 6,0 | Rußbildung |
| 10. Stahl, Werkstoff Nr. 1.4841 | 22 | 16 | 10,6 | 1,1 | |
| 11. Stahl, Werkstoff Nr. 1.4541 | 12 | 18 | 12 | 4,2 → 2,0 | |
| 12. Rein-Nickel, poliert | 16 | 11 | 22,4 | 1,0 | Rußbildung |

Tabelle 2 (Fortsetzung)

Erfindungsgemäße Beispiele

| Rohrmaterial | Laufzeit (h) | CO-Zunahme (Vol.-%) | Gehalt in Vol.-% an $CH_4$ | $C_2H_4$ | Bemerkungen |
|---|---|---|---|---|---|
| 13. Aluchrom® | 250 | 0,5 | 16 | 6,2 | nach 89 h spontaner Ethylenabfall |
| 14. dito 13; sulfidiertes Gasgemisch | 250 | 0 | 14,8 | 5,7 | |
| 15. Aluchrom® W | 92 | 0 | 14,6 | 7,1 | |
| 16. Kanthal® DSD | 250 | 0 | 12,2 | 5,25 | |
| 17. Inconel® 601 mit Al belegt | 250 | 1 | 16 | 6,9 | |
| 18. Armco 18 SR® | 250 | 2,6 | 16,8 | 6,6 | |
| 19. dito 18; mit Al belegt | 250 | 1,5 | 16,8 | 7,2 | |
| 20. Stahl, Werkstoff Nr. 1.4571, mit Al und Cu belegt | 250 | 0 | 15,6 | 6,2 | |
| 21. Stahl, Werkstoff Nr. 1.4571, mit Al belegt | 250 | 0 | 16,4 | 6,7 | |
| 22. Stahl, Werkstoff Nr. 1.4571, mit Cu belegt | 250 | −3,3 | 14,3 | 5,7 | |

**Patentansprüche**

1. Verfahren zur Herstellung von Ethylen durch Hydropyrolyse eines Kohlenwasserstoffe, Wasserstoff, Kohlenmonoxid, Kohlendioxid und Wasserdampf enthaltenden Gasgemisches in einer Reaktionszone bei Temperaturen von über 800°C bis zu 1000°C, dadurch gekennzeichnet, daß das Gasgemisch in der Reaktionszone einen Druck von weniger als 5 bar aufweist und daß die vom Gasgemisch berührten metallischen Wände der Reaktionszone mindestens an ihrer Oberfläche Aluminium und/oder Kupfer enthalten.

2. Verfahren nach Anspruch 1, dadurch gekennzeichnet, daß das Gasgemisch einen Druck von 1,5 bis 4 bar aufweist.

3. Verfahren nach Anspruch 1 oder 2, dadurch gekennzeichnet, daß die metallischen Wände der Reaktionszone aus einer aluminium- und/oder kupferhaltigen Stahlsorte bestehen.

4. Verfahren nach einem der Ansprüche 1 bis 3, dadurch gekennzeichnet, daß auf die metallischen Wände der Reaktionszone Aluminium und/oder Kupfer aufgetragen sind.

5. Verfahren nach Anspruch 4, dadurch gekennzeichnet, daß das Auftragen von Aluminium und/oder Kupfer auf die metallischen Wände über die Gasphase mit Hilfe von Halogenverbindungen als Transportmittel bewirkt wird.

6. Verfahren nach Anspruch 4, dadurch gekennzeichnet, daß das Auftragen von Aluminium durch Inberührungbringen der metallischen Wände mit einer Schmelze aus Aluminium bewirkt wird.

7. Verfahren nach einem der Ansprüche 1 bis 6, dadurch gekennzeichnet, daß dem Gasgemisch gasförmige Schwefelverbindungen zugesetzt sind.

**Claims**

1. Process for making ethylene by subjecting a gas mixture containing hydrocarbons, hydrogen, carbon monoxide, carbon dioxide and steam to hydropyrolysis in a reaction zone at temperatures higher than 800°C up to 1000°C, characterized in that the gas mixture presents, in the reaction zone, a pressure of less than 5 bars and in that the metallic walls of the reaction zone which come into contact

with the gas mixture contain aluminum and/or copper in at least their surface portions.

2. Process as claimed in claim 1, wherein the gas mixture presents a pressure of 1.5 to 4 bars.

3. Process as claimed in claim 1 or 2, wherein the metallic walls of the reaction zone are made up of a steel grade containing aluminium and/or copper.

4. Process as claimed in any of claims 1 to 3, wherein the metallic walls of the reaction zone have aluminum and/or copper applied thereto.

5. Process as claimed in claim 4, wherein the aluminum and/or copper is (are) applied to the metallic walls via the gas phase with the use of halogen compounds as transporting agents.

6. Process as claimed in claim 4, wherein the aluminum is applied to the metallic walls by contacting the latter with a melt of aluminum.

7. Process as claimed in any of claims 1 to 6, wherein the gas mixture is used in further admixture with gaseous sulfur compounds.

**Revendications**

1. Procédé de préparation d'éthylène par hydropyrolyse d'un mélange gazeux contenant des hydrocarbures, de l'hydrogène, du monoxyde de carbone, du dioxyde de carbone et de la vapeur d'eau dans une zone de réaction à des températures supérieures à 800° C jusqu'à 1000° C, caractérisé en ce que le mélange gazeux présente, dans la zone de réaction, une pression inférieure à 5 bars et en ce que les parois métalliques de la zone de réaction en contact avec le mélange gazeux contiennent de l'aluminium et/ou du cuivre, au moins à leur surface.

2. Procédé selon la revendication 1, caractérisé en ce que le mélange gazeux présente une pression de 1,5 à 4 bars.

3. Procédé selon la revendication 1 ou 2, caractérisé en ce que les parois métalliques de la zone de réaction consistent en une sorte d'acier contenant de l'aluminium et/ou du cuivre.

4. Procédé selon l'une des revendications 1 à 3, caractérisé en ce que les parois métalliques de la zone de réaction sont revêtues d'une couche d'aluminium et/ou de cuivre.

5. Procédé selon la revendication 4, caractérisé en ce que les couches d'aluminium et/ou de cuivre sont appliquées sur les parois métalliques en phase gazeuse à l'aide de composés halogénés comme agents de transport.

6. Procédé selon la revendication 4, caractérisé en ce que l'on applique la couche d'aluminium par mise en contact des parois métalliques avec une masse fondue d'aluminium.

7. Procédé selon l'une des revendications 1 à 6, caractérisé en ce que l'on utilise le mélange gazeux en association avec des composés gazeux de soufre.